# EUROPEAN PATENT SPECIFICATION

(11) **EP 3 166 692 B1**
(45) Date of publication and mention of the grant of the patent: **06.11.2019**
(21) Application number: 15736497.7
(22) Date of filing: 10.07.2015
(51) Int. Cl.: A61Q 11/00, A61K 8/44, A61K 8/24

(54) **ORAL CARE FORMULATION SYSTEM PROVIDING AMORPHOUS CALCIUM PHOSPHATE**
AMORPHES CALCIUMPHOSPHAT BEREITSTELLENDES MUNDPFLEGEFORMULIERUNGSSYSTEM
SYSTÈME DE FORMULATION DE SOINS ORAUX DÉLIVRANT DU PHOSPHATE DE CALCIUM AMORPHE

(30) Priority: 11.07.2014 EP 14176834
(43) Date of publication of application: 17.05.2017
(73) Proprietor: Koninklijke Philips N.V., 5656 AE Eindhoven (NL)
(72) Inventor: MOHAN, Veena, NL-5656 AE Eindhoven (NL)
(74) Representative: Marsman, Albert Willem
(86) International application number: PCT/EP2015/065835
(87) International publication number: WO 2016/005559

(56) References cited:
- WO-A1-2006/056013
- WO-A1-2009/099452
- WO-A1-2009/099455
- WO-A1-2010/134904
- WO-A2-2009/100263
- DE-A1- 2 543 489
- US-A- 5 037 639
- US-A1- 2005 175 959

## Description

### FIELD OF THE INVENTION

The invention is in the field of oral care formulations that serve as a source for the delivery of amorphous calcium phosphate to teeth. More particularly, the invention pertains to a two-component whitening gel or varnish providing a source of amorphous calcium phosphate, as defined in the appended claims.

### BACKGROUND OF THE INVENTION

Erosion is becoming important in the management of the long-term health of dentition. Demineralization of dental structures leads to caries, decayed dentin, cementum, and/or enamel and tooth sensitivity. This can be caused by several factors including the aggressive oxygenation from bleaching agents. Unlike enamel and cementum the dentine is transversed by numerous tubules. Dentin is susceptible to rapid demineralisation because of the presence of organic matrix, more open space tubules and more accessible for ion diffusion. The tubule walls are comprised of calcified matrix of the dentine and the tubule space is filled with fluid derived from pulp tissue fluid (dentinal fluid) and serum. The matrix mineral is comprised mainly of calcium phosphate salt, Hydroxyapatite, which is poorly soluble at neutral and alkaline pH and progressively more soluble at acidic pH.

Ideally, dentine tubules have naturally formed smear plugs. Dentinal hypersensitivity results when the enamel or dentine is lost. When this is lost, a stimuli applied to dentin can increase the flow of dentine tubular fluid within the tubules. The fluid that fills the narrow dentinal tubules enable cold, tactile, evaporative and osmotic stimuli to be transmitted through dentine to the pulp in the form of fluid movement. This movement of dentinal fluid is sensed as sharp pain of short duration as a result of the stimulation of nerve fibres. This creates pressure on the pulp, resulting in deformation of the cell membranes of nerve endings.

Cementum is easier to breach than enamel and this cannot happen until there is gingival recession and exposure of the root surface to the oral environment. The loss of cementum can cause from dental caries, acidic agents, vigorous brushing, abrasive toothpaste, procedures performed by dentist or hygienist in dental office.

While vital bleaching with a peroxide gel is generally recognized as both safe and effective, transient dental hypersensitivity is a common, unpleasant side effect of the treatment. The etiology of bleaching related tooth sensitivity is neither well understood nor easily measured. According to the hydrodynamic model, peroxide solutions introduced into the oral environment contact available dentinal surfaces and cause retraction of odontoblastic processes, resulting in rapid fluid movement inside dentinal tubules. This ultimately manifests in stimulation of mechanoreceptors at the pulp periphery. As a result patients can feel a clinically evident painful sensation when such teeth are exposed to cold or pressure or even when they are at rest. In order to reduce this discomfort felt during the bleaching process, manufacturers uses potassium nitrate as de sensitising agent. However this does not provide any prolonged effect.

Many of the products in market for tooth sensitivity contain agents such as Fluoride, bioglass, casein phosphor peptide, potassium oxalate etc. The use of Fluoride causes the products to be somewhat unstable and slow acting and can cause significant tooth discolouration. A newer approach to dentifrice based desensitising may use Amorphous Calcium Phosphate (ACP). This is originally developed by the American Dental Association Foundation to remineralise teeth and reverse early enamel carious lesions. ACP rapidly obliterates the dentinal tubules by rapid precipitation of calcium phosphate crystals on the surface and also inside the dentinal tubules. It may also have the ability to directly depolarize nerve endings however this is not proved yet.

Amorphous Calcium Phosphate (ACP) phases are one of the most frequent forms of Calcium Phosphate minerals in biological organisms. Under oral conditions, ACP has the highest rates of formation and dissolution among all calcium phosphates, exception of the highly acidic monocalcium phosphate. The ACP phase is an intermediate phase in the preparation of several calcium phosphates by precipitation.

ACP delivery may involve a two phase delivery system to keep the calcium and phosphorous components from reacting with each other before application. When two salts are mixed, they rapidly from amorphous calcium phosphate on the surface of tooth. This precipitated ACP can then readily dissolve into saliva and be available for tooth remineralisation.

A background reference in this respect is WO 2006/053207. Herein a two-component dental whitening system is disclosed. Therein one of the components comprises at least one peroxide and the other component comprises an Activator Gel comprising at least one transition metal salt. The Activator Gel catalyses the decomposition of the peroxide. The system may comprise precursors for ACP, whereby a source of calcium ions is present in one of the components, and a source of phosphoric ions in the other.

The release of ions (e.g., calcium, and preferably calcium and phosphate ions) into the oral environment is known to enhance the natural remineralising capability of dental structures. It is believed that enhanced remineralisation may be a useful supplement to, or even an alternative to, traditional dental restorative methods. Remineralisation of the dentin to obstruct the tubules is an ideal way to treat dentin hypersensitivity in exposed roots. To be clinically practical, treatments must re-mineralise rapidly. Here, higher concentrations of Calcium and Phosphate are used to increase the diffusion rates of ions and hence the remineralisation rates. (see e.g. US5037639)

A drawback of dental whitening products, such as disclosed in WO 2006/053207, is that they contain relatively low concentrations of ACP precursors and hence the relatively long duration required for remineralisation process and occlusion of dentine tubules. It is not straightforward, however, to just increase the concentration of the precursors to increase the reaction rate, as this results in the formation and precipitation of calcium phosphate crystals, without any benefit of amorphous calcium phosphate being given. It is thus desired to provide a formulation that makes it possible to increase the concentrations of ACP precursors, without rapid crystallization.

Further, the aforementioned background art relates to a gel based system. It would be desired to also be able to provide ACP, particularly in relatively high concentrations, via a varnish-type system, preferably a tooth whitening varnish. Whilst gel-based systems can well be water-based, varnish systems are typically based on ethanol or other vaporizable anhydrous carrier liquids. Particularly from such solvents, the *in situ* formed ACP will be prone to untimely precipitation, or even crystallization. It is thus desired to provide an ethanol-based varnish formulation that comprises ACP precursors, but which does not lead to untimely precipitation of ACP and, preferably, also in the event of increased concentrations of ACP precursors.

### SUMMARY OF THE INVENTION

In order to better address one or more of the foregoing desires, the invention presents, in one aspect, an oral healthcare system comprising components for the delivery of amorphous calcium phosphate, wherein a first component comprises a calcium salt and a second component comprises a source of phosphoric ions selected from the group consisting of orthophoshoric acid and phosphate salts, said components comprising a liquid carrier comprising a polar solvent, wherein either or both of the components comprise one or more amino acids.

In another aspect, the invention concerns the use of one or more amino acids as an anti-crystallization agent for amorphous calcium phosphate, formed in the oral cavity of a subject, as a result from combining a composition comprising a calcium salt and a composition comprising a source of phosphate ions selected from the group consisting of orthophoshoric acid and phosphate salts, said compositions comprising a liquid carrier selected from the group consisting of water, ethanol, and mixtures thereof, wherein the one or more amino acids are present in said compositions.

### DETAILED DESCRIPTION OF THE EMBODIMENTS

In a broad sense, the invention is based on the judicious recognition that amino acids are capable of preventing the rapid crystallization of calcium phosphate, upon its *in situ* formation in the environment of the oral cavity. Said *in situ* formation, and the beneficial effects of thus formed amorphous calcium phosphate (ACP), have been well evidenced in the art. See, *inter alia,* the following references which regard the possible benefits of ACP in dental care: Yates et al. (1998), Journal of Clinical Periodontology 25:687-692; Giniger et al 2005, The Journal of the American Dental Association 136: 383-392; Tung et al. (1993), Journal of Endodontics 19 :383-387.

It is to be noted that the invention is specifically directed to oral healthcare systems comprising a source of calcium ions and a source of phosphoric ions in separate components. This is not to be confused with possibly existing composition, wherein ACP is present as such, and may therein be in a stabilized form. See, e.g., US 2006/292092, wherein stabilized ACP is present as a fixed component. The stabilized ACP thereby is in fact a modified ACP (e.g. metal or silicon modified). The technical problems addressed by the invention particularly relate to crystallization of ACP as a result of its *in situ* formation from combining two liquid components. This problem plays a role particularly if it is desired to enable providing increased amounts of ACP. Further, in ethanol-based formulations it is an even stronger challenge to effectively deliver ACP, from precursor compositions, to the oral cavity.

The invention, in one aspect, is a system for oral healthcare. The term system indicates that the invention in some embodiments does not come as a single composition, but as a kit of parts, more particularly comprising at least two components (sometimes also indicated as "phases"). These components, together, are to act as a source of ACP. Preferably, the oral healthcare system is for delivery of amorphous calcium phosphate, preferably to an oral cavity, for example to teeth. This includes providing calcium ions and phosphoric ions in solution. Hereinafter, these components are sometimes indicated as the "calcium component" (viz. the component comprising a source of calcium ions) and the "phosphor component" (viz. the component comprising a source of phosphoric ions selected from the group consisting of phosphoric acids and phosphoric salts).

The components comprise a liquid carrier comprising a polar solvent, including mixtures of polar solvents. Polar solvents are known to be classified in different ways. Here a classification based on dielectric constant is adopted. Polar solvents include polar aprotic solvents and polar protic solvents. Preferred solvents used in the invention are polar protic solvent. Particularly interesting polar protice solvents are selected from the group consisting of water, acetic acid, C₂-C₁₀ mono-alcohols, C₂-C₁₀ polyols, and mixtures thereof. Examples of suitable alcohols include ethanol, n-propanol, and isopropanol, and combination thereof. In particularly interesting embodiments, acetic acid is employed as a co-solvent, i.e. preferably in combination with one or more other solvents.

The calcium component preferably comprises 0.01 to 50 wt.% of the calcium salt. Suitable sources of calcium ions include, but are not limited to, calcium nitrate, calcium carbonate, calcium chloride, calcium hydroxide, calcium acetate. A particularly interesting source of calcium is calcium nitrate. The calcium component may for instance contain calcium salts dissolved in solution, for example in water.

The phosphor component preferably comprises an amount of 0.1 to 20 wt.% of the phosphor source. Suitable sources of phosphate are selected from the group consisting of orthophoshoric acid and phosphate salts. Orthophosphoric acid, also known as phosphoric(V)acid has the molecular formula H₃PO₄. Phosphate salts are the salts of orthophosphoric acid. These salts can be monobasic (salts of H₂PO₄, preferably sodium phosphate monobasic) dibasic (salts of HPO₄= , i.e., sodium phosphate dibasic), or tribasic (salts of PO₄³⁻). Other sources of phosphate include tetrapotassium pyrophosphate or sodium pyrophosphate. In general any phosphate source can be used without impairing the activity of other components in the formulation. The phosphate salt may further act to adjust the pH of the composition. Other alkali and earth alkali phosphates, such as potassium phosphates, can also be used. A preferred source according to the present invention is orthophosphoric acid. This choice deviates from the common belief that the phosphate source should itself be a phosphate, and it has turned to work very effectively. The phosphoric component may for instance contain phosphate salts dissolved in a solution, for instance in water.

In all of the components in the system of the invention, each independently, mixtures of calcium sources, mixtures of phosphate sources, and mixtures of amino acids can be used.

Preferably, the system according to the invention provides a higher concentration of ACP than existing commercial systems.

In accordance with the invention, one or more amino acids are present in the calcium component, or in the phosphor component, or in both of these components. Preferably, the amino acid is present in the phosphor component. In an interesting embodiment, an acidic or neutral amino acid is present in the phosphor component.

Amino acids are known to the skilled person. Amino acids are organic compounds carrying both an amino group (e.g.,NH₂) and a carboxylic acid group (-COOH), and are further differentiated depending on their side-chains.

Basic amino acids are defined as amino acids that, at neutral pH (i.e., pH 7), have basic side chains. Correspondingly, acidic amino acids have acidic side chains as neutral pH, and neutral amino acids have a neutral side-chain at neutral pH. Natural as well as non-natural amino acids are included, but natural amino acids are preferred.

Basic amino acids are preferred. Preferably these are natural amino acids, i.e. the amino acids for use in the present invention are preferably selected from the group consisting of arginine (Arg), lysine (Lys), histidine (His), and mixtures thereof.

The amino acids are for example not phosphorylated. The amino acids for example do not bear a hydroxyl group.

The amino acid or acids are present in the composition in an amount ranging from 0.5 wt.% to 10 wt.%, preferably 2 wt.% to 8 wt %, based on total weight of the component in which they are included, and/or on the total weight of the components together.

Either or both of the components, or any further components that are optionally comprised in the system of the invention, can have other oral care active agents present. Such agents are preferably selected from the group consisting of dental whitening agents, antiplaque agents, anti-tartar agents, anti-gingivitis agents, anti-bacterial agents, anticaries agents, and combinations thereof.

A preferred anti -caries agent is fluoride. The fluoride can be provided as a separate component, but preferably is comprised in either the calcium component or the phosphor component. Most preferably, fluoride is present in the phosphor component.

Suitable fluoride sources include sodium fluoride, stannous fluoride, sodium monofluorophosphate, zinc ammonium fluoride, tin ammonium fluoride, calcium fluoride, cobalt ammonium fluoride potassium fluoride, lithium fluoride, ammonium fluoride, zinc ammonium fluoride, tin ammonium fluoride, calcium fluoride, cobalt ammonium fluoride, water soluble amine hydrofluorides, or mixtures thereof. The fluoride-containing component preferably comprises the fluoride source in an amount of at least 0.001 %, more preferably, from 0.01 to 12%, and most preferably, from 0.1 to 5% by weight of the total system.

Other possible oral healthcare active agents that can be included in either or both of the components are, e.g., antibacterial agents. These include, for example, phenolics and salicylamides, and sources of certain metal ions such as zinc, copper, silver and stannous ions, for example in salt form such as zinc, copper and stannous chloride, and silver nitrate. These are present in art-known small quantities when used. In addition, optional additives can be present in either or both of the components. These include, e.g., humectants, flavourings, colouring agents, anti-plaque agents, anti-staining compounds, pH adjusting agents, excipients such as emollients, preservatives, other types of stabilizers such as antioxidants, chelating agents, tonicity modifiers (such as sodium chloride, mannitol, sorbitol or glucose), spreading agents, and water soluble lubricants, such as propylene glycol, glycerol or polyethylene glycol. The concentration of each may easily be determined by a person skilled in the art.

Humectants include water, polyols, such as glycerol, sorbitol, polyethylene glycols, propylene glycols, hydrogenated partially hydrolysed polysaccharides and the like. A single humectant or a combination is also contemplated. They are generally present in amounts of, for example, up to about 85%, more for example, from about 15% to about 75% of the formulation.

Additional de-sensitizing agents may also be used. Suitable desensitizing agents, if added, may include, for example, alkali nitrates such as potassium nitrate, sodium nitrate and lithium nitrate; and other potassium salts such as potassium chloride and potassium bicarbonate.

The formulation may contain at least one at least one alkali metal salt having desensitizing effect and an amorphous calcium and/or strontium phosphate. In addition to these de-sensitizing agents amorphous calcium compounds such as amorphous calcium phosphate (ACP), amorphous calcium phosphate fluoride (ACPF) and amorphous calcium carbonate phosphate (ACCP) calcium carbonate phosphate (ACCP), amorphous calcium carbonate phosphate fluoride (ACCPF) or mixtures thereof may also be used. These amorphous compounds are disclosed in U.S. Patent Nos.5,037,639, 5,268,167, 5,437,857, 5,562,895, 6,000,341, and 6,056,930.

In addition to amorphous calcium compounds, amorphous strontium compounds such as amorphous strontium phosphate (ASP), amorphous strontium phosphate fluoride (ASPF), amorphous strontium calcium phosphate (ASCP), amorphous strontium calcium carbonate phosphate (ASCCP), amorphous strontium carbonate phosphate fluoride (ASCPF), amorphous strontium calcium carbonate phosphate fluoride (ASCCPF) and mixtures thereof may also be used.

The system of the invention, particularly varnish based systems, can be provided as a stand-alone desensitizing treatment, regardless of the origin of the dental sensitivity to be treated. This includes, e.g., sensitivity from gum recession, root exposure, post root planning, etc. The sensitivity to be treated also includes sensitivity caused by teeth whitening procedures, such as with hydrogen peroxide. In that case, the system of the invention does not itself contain a bleaching agent such as hydrogen peroxide. In an interesting embodiment, the system for use as a stand-alone treatment comprises additional remineralization and/or desensitizing agents, particularly sodium fluoride, potassium nitrate, or a combination thereof.

In a further interesting embodiment, the system of the invention itself comprises, as an oral care active agent, a tooth whitening agent. Thereto the system of the invention may include any commercial whitening composition including Zoom(R)!gel, Zoom(R)! Turbo, Zoom(R) 2 gel, BriteSmile(R) Procedure Gel; BriteSmile(R) Take Home Gel; and others, all available from Discus Dental, Inc. or BriteSmile Professional, Inc., Culver City, Calif. Some of these gels are capable of whitening without the assistance of heat and/or light. Examples include NiteWhite(R), DayWhite(R), NiteWhite(R) Turbo, NiteWhite(R) ACP, Nitewhite(R) ACP Turbo, DayWhite(R) ACP, WhiteSpeed(R) JumpStart, Zoom Weekender(R), BriteSmile(R)-to-go or BTG Pen, also available from Discus Dental, Inc. or BriteSmile Professional, Inc., Culver City, Calif. Some of these are described in more detail in U.S. Pat. Nos. 5,908,614, 6,576,227; 6,221,341; 6,488,914 5,922,307; 6,331,292; 6,986,883; U.S. publication nos. 2003/211055; 2004/0101497; 2005/0008584; 2005/0026107; 2004/0146467; U.S. application Ser. No. 11/271,283 entitled, "Dental Whitening Systems" filed on Nov. 9, 2005; Ser. No. 11/271,412 entitled, "Dental Whitening Compositions" filed Nov. 9, 2005; Ser. No. 11/288,504 entitled, "Dental Whitening Compositions," filed Nov. 28, 2005, the contents of all of these are incorporated herein by reference.

Some of these whitening gels may be light and/or heat activated gels and include those described in U.S. Pat. Nos. 5,645,428; 5,713,738; 6,258,388; 6,361,320; 6,162,055; 6,343,933; 6,416,319; 6,958,144; U.S. publication nos. 2002/0137001; 2002/0141951; 2003/0198605; 2003/0089886; 2005/0084825; 2005/0265933; 2005/0048434; U.S. application Ser. No. 11/271,283 entitled, "Dental Whitening Systems" filed on Nov. 9, 2005.

In a preferred embodiment of a system according to the invention, the components together form a two component dental whitening system. Particularly, one of the components therein comprises at least one peroxide and the other component comprises an Activator Gel, in some embodiments comprising at least one transition metal salt. The Activator Gel catalyses the decomposition of the peroxide. The whitening system of this embodiment can be provided in accordance with the disclosure of WO 2006/053207). In accordance with the invention, it is preferred that one of the components of the whitening system also serves as the calcium component, and the other component of the whitening system also serves as the phosphor component. Preferably, the peroxide is present in the phosphor component.

The system of the invention is in the form of a gel or a varnish to be applied onto one or more teeth of a subject. The invention is suitable for gels or varnish for both personal and professional oral care. The latter is of importance particularly for professional whitening systems, which use substantially higher concentrations of whitening agents and may therefore benefit also from increased concentrations of ACP.

In dental care, such gels and varnishes are specifically designed to stay on the teeth for a much longer time than in conventional dental care products such as toothpastes or mouthwash. These products essentially are not only used quickly (typically 2 minutes for toothpaste and half a minute for mouthwash), but are also removed thereafter relatively quickly either by spitting out or by dilution from saliva. The present invention particularly is directed to such products as gels and varnishes that are intended to be applied, with some adhesiveness, on to the teeth.

Gels according to the invention are generally water-based, and comprise a gellable polymer. Typical polymers are ethylene oxide copolymers, for example, those including both a hydrophilic component and a hydrophobic component, including copolymers of ethylene oxide and propylene oxide. The copolymers may be block copolymers of propylene oxide (hydrophobic component), and ethylene oxide (hydrophilic component). The propylene oxide block is generally sandwiched between two ethylene oxide blocks. Examples include Pluronic F-127, P-84, F-108, F-98, F-88, F-87 and mixtures thereof, available from BASF Corporation (North Mount Olive, N.J., USA). Preferred gelling agents are triblock copolymers comprising a central hydrophobic block of polypropylene glycol flanked by two hydrophilic blocks of polyethylene glycol, e.g. as available under the trade name Poloxamer 407, or hydroxypropylcellulose, e.g. obtainable commercially as Klucel GF. The gelling agent, particularly said polymers, are generally present in a concentration range from 1-40% (w/w) of the total system.

Other gelling agents which may be used in the preparation of gels, particularly of whitening gels include, for example, cellulosic gums fumed silica, for example, CAB-O-SIL fumed silica provided by Cabot Corporation, and emulsifying waxes such as Polawax (emulsifying wax NF) or Crodafos CES (cetearyl alcohol (and) dicetyl phosphate (and) ceteth-10 phosphate), provided by Croda, Inc., and mixtures thereof, in amounts to provide a stable gel a lower viscosity gelling agent or thickener may be used. They are not as likely to inhibit the availability of active peroxides to the same extent as a higher viscosity gelling agent. The viscosity is for example, generally less than about 10000 cps, more for example, less than about 8000 cps, and even more for example, less than about 5000 cps.

Varnishes are generally based on a vaporizable (volatile) liquid carrier, typically ethanol, isopropanol, or other C₂-C₁₀ mono-alcohols or C₂-C₁₀ polyols, possibly together with acetic acid. The varnishes typically comprise a polymer component, which after the evaporation of the liquid carrier will serve as a carrier or matrix so as to allow retaining the desired components onto the surface of a subject's teeth. Suitable polymers are, e.g., copolymers of ethyl acrylate, methyl methacrylate and a low content of methacrylic acid ester with quaternary ammonium groups, such as commercially obtainable from Evonik as Eudragit RLPOor RSPO. Another suitable polymer is polyvinyl pyrrolidone, e.g. as obtainable from BASF. Various grades of these, other, polymers can be used. Preferably these polymers render the system visco-elastic, so as to promote adhesion onto the teeth. Polymers are generally present in a concentration range from 1-40% (w/w) of the total system. Preferably, the polymer is Eudragit RLPO or RSPO in combination with Eudragit L100-55 (methacrylic acid - methyl methacrylate copolymer).

The system of the invention can be used just as according to known practice for two-component gels and varnishesd, such as the whitening system described in WO 2006/053207. Typically, immediately before use, the components are mixed together (for practical reasons usually in a 1:1 ratio, although conceivably the system could also be tuned to using different volumes of either component, with the calcium ion and phosphate contents adjusted appropriately). This can be done, e.g., by actuating a syringe or a container depending on how the components are provided. The admixed gel or varnish is applied to the surface of the teeth directly from the syringe or container, by means of, e.g., a dental tray (typically, as in whitening gels or varnish, a dental bleaching tray).

In one embodiment of the invention, the two components of the system may be provided in a two barrel syringe. In one aspect, the syringe may be provided with a dispensing tip. In another aspect, the dispensing tip may be adapted for foaming. In a further aspect, the tip may include a mixer.

In another embodiment of the invention, the two components of the system may be provided in a container having separate compartments for the components. In one aspect, the container may be provided with a dispensing pump.

The presence of two component system also helps in stabilizing the whitening agent from degradation at higher pH and keeping the overall pH of the formulation optimum for use in the oral cavity.

The ACP precursors are generally present separately, for example, a first component may include a source of phosphate and a second component may include a source of calcium or strontium. When the two components are mixed, the source of phosphate and the source of calcium strontium or mixture may combine to form amorphous calcium phosphate, which when applied to teeth, may precipitate onto the surface of the teeth where it may be incorporated into hydroxyapatite, assisting in remineralization of the tooth enamel, as well as decreasing sensitivity, as noted above.

Generally, a system comprising at least two components may provide these components for instance as separate parts of a kit of parts, as a packaging containing separate compartments, and/or as a multiphase composition comprising different phases comprising the components. Such a multiphase composition is preferably fluid. Preferably, the components have a different composition.

Preferably, the concentration of calcium ions in the calcium component is at least 2 times higher, more preferably at least 10 times higher than in the phosphor component. Preferably, the concentration of phosphoric ions is at least 2 times higher, more preferably at least 10 times higher in the phosphor component than in the calcium component.

Even though the source of calcium or strontium is kept separately from the source of phosphate, a separate container or compartment is not the only way to effect separation. Separation may also be effected by means of distance, or a partition which may involve encapsulating the source of calcium or strontium or amino acid in one or multiple capsule, layer or layers or an immobilized medium, generally referred to as a component, and the source of phosphate in another capsule, layer or an immobilized medium, also generally referred to as a component.

The gel could be dispensed from syringe or other related embodiments and then applied evenly on teeth using a paint brush or other applicators.

The varnish could be dispensed from syringe or other related embodiments and then applied evenly on teeth by brushing, spraying, or with a wipe. When applied on the teeth, may be allowed to cure or in some embodiments at least one of heat, an air jet, and light may be applied to speed up the process. The applicator may also include a fan to reduce curing time.

Lip retraction may be used to keep the lips from coming into contact with the compositions as they cure. Curing is used herein to describe any process by which the composition forms an intact layer on the teeth which is capable of remaining on the teeth throughout the whitening process. Vibration maybe used during curing to improve the smoothness of the layer. Soft tissue in the mouth, such as the gums, maybe protected, prior to applying the compositions with a layer of a suitable material (e.g., a soft and smooth material).

The composition may be applied by a dental professional, such as a dentist, or by the wearer. For example, the composition may be applied to the teeth using an applicator, such as a pen, brush, piece of foam, cloth applicator, dental tray, or to two-compartment syringe to form the first layer.

In other embodiments, the composition may be inserted into an applicator, such as into the dental tray, which is positioned adjacent the teeth and then removed, for example, after partial drying/curing of the composition. The first composition 10 maybe applied to the teeth at a thickness t of for example, from 50-500µm, such as from 50-300µm, e.g., about 200µm. In the some embodiments wherein the oral care agent is present in the form of particles, the film is greater in thickness than the average diameter of the particle, for example, at least twice or at least three times the average diameter of the particles. This allows the varnish to be smooth to the touch, when cured. The curing/hardening may be performed with light, air, moisture, solvent evaporation, or a combination of these

Without wishing to be bound by theory, the inventors believe that the amino acids serve to better retain, in the environment of the oral cavity, the amorphous character of ACP formed *in situ.* This represents a novel use of amino acids, particularly basic amino acids. In another aspect, therefore the invention concerns the use of one or more amino acids, preferably basic amino acids as an anti-crystallization agent for amorphous calcium phosphate, formed in the oral cavity of a subject, as a result from combining a composition comprising a calcium salt and a composition comprising a source of phosphate ions selected from the group consisting of orthophoshoric acid and phosphate salts, said compositions comprising a liquid carrier selected from the group consisting of water, ethanol, and mixtures thereof, wherein the one or more amino acids are present in said compositions. The foregoing use can be realized generally with reference to the embodiments of the invention as described hereinbefore.

The system of the invention serves to administer amorphous calcium phosphate into the oral cavity. The system of the invention is capable of leading to the occlusion of dental tubules and/or the deposition of remineralising agents on dental enamel, particularly in a shorter treatment period than with the current standard of care treatments. In this respect, the invention also includes, in a further aspect, a composition comprising amorphous calcium phosphate for use in a dental treatment, particularly a desensitizing treatment, more particularly for the occlusion of dental tubules, and/or the deposition of remineralising agents on dental enamel. In said use, the composition is applied by bringing together the components of a system according to one or more of the embodiments of the invention described hereinbefore, in the oral cavity of a subject. Similarly, the invention also concerns a method for the foregoing dental treatments.

### Example 1

Model formulations for a two-component ACP gel are provided. The components have the compositions as outlined below.

**Component 1**

| **Materials** | **Amount (g)** |
|---|---|
| Calcium nitrate | 15 |
| Arginine | 8.00 |
| Water | 77 |

**Component 2**

| **Materials** | **Amount (g)** |
|---|---|
| Sodium phosphate mono basic | 3.1414 |
| Sodium phosphate di basic | 2.6902 |
| Arginine | 8.00 |
| Water | 86.1684 |

The formulation resulting from combining the components did not produce any crystals after one hour period in the presence of amino acid.

The formulation was applied on prepared bovine dentine sample and incubated at 22°C for 30 minutes to observe tubule occlusion. After 15 minutes of application, the dentine tubules were not occluded by the formulation. However multiple application of formulation for another 30 minutes showed occlusion of tubules under CLSM (images based on Confocal Laser Scanning Microscopy).

### Reference Example (formulation without amino acid)

**Component 1**

| **Materials** | **Amount (g)** |
|---|---|
| Calcium nitrate | 15 |
| Water | 85 |

**Component 2**

| **Materials** | **Amount (g)** |
|---|---|
| Sodium phosphate mono basic | 3.1414 |
| Sodium phosphate di basic | 2.6902 |
| Water | 94.1684 |

In a test as in Example 1, viewed at 30 minutes, 45 minutes and 60 minutes, no effect was seen using CLSM.

### Example 2

This example is similar to Example 1, with increased concentrations of the ACP precursors.

**Component 1**

| **Materials** | **Amount (g)** |
|---|---|
| Calcium nitrate | 35.42 |
| Arginine | 8.00 |
| Water | 56.58 |

**Component 2**

| **Materials** | **Amount (g)** |
|---|---|
| Sodium phosphate mono basic | 7.10 |
| Sodium phosphate di basic | 6.00 |
| Arginine | 8.00 |
| Water | 78.9 |

This formulation too was tested on bovine dentine to observe the occlusion of dentine tubule. Based on CLSM images taken at various times (confocal laser scanning microscopy) the dentine tubules were occluded completely by the applied formulation containing the increased concentration of calcium and arginine after 30 minutes treatment period.

### Example 3

This example is equal, in amounts to Example 1, with the amino acid lysine substituted for the amino acid arginine in component 1.

This formulation was tested directly on bovine dentine and kept at 37°C for total treatment time of 30 minutes. After 15 minutes treatment the samples were washed with distilled water to remove unbound layer from the surface. It was then air dried for 2-3 minutes to observe the occlusion of tubules under CLSM, with complete occlusion in after 30 minutes shown.

### Example 4

This example is equal, in amounts, to Example 1, with the amino acid composition in both components being 4 g of lysine and 4 g of arginine.

Treatment of formulation on bovine dentine showed complete occlusion of the tubules after 30 minutes.

### Example 5

**Component 1**

| **Materials** | **In grams** |
|---|---|
| Calcium nitrate | 15 |
| Arginine | 2 |
| Lysine | 6 |
| Water | 77 |

**Component 2**

| **Materials** | **In grams** |
|---|---|
| Sodium phosphate mono basic | 3.1414 |
| Sodium phosphate di basic | 2.6902 |
| Lysine | 8 |
| Water | 86.1684 |

This example is equal, in amounts, to Example 4, with the amino acid composition in both components being 6 g of lysine and 2 g of arginine.

Compared to other formulations, it worked faster, and occluded almost all the tubules with single 15 minutes treatment period.

### Example 6

Model formulations for a two-component ACP varnish are provided. The components have the compositions as outlined below.

**Component 1**

| **Materials** | **Amount (g)** |
|---|---|
| Calcium nitrate | 28 |
| Ethanol | 72 |

**Component 2**

| **Materials** | **Amount (g)** |
|---|---|
| Arginine | 4 |
| Lysine | 8 |
| Ethanol/acetic acid | 70 (63/7) |
| Sodium phosphate mono basic | 2 |
| Sodium phosphate di basic | 4 |
| Acetic acid (99.7%) | 12 |

The occlusion of dentine tubules with this formulation was investigated. The dentine tubules were imaged by CLSM before doing the treatment and the treatment was carried out for continuous 30 minutes twice. Most of the tubules were partially occluded with 60 minutes application. Also the occlusions were not washed away during their storage in phosphate buffered saline.

### Example 7

This example concerns a model whitening varnish formulation having a concentration of calcium nitrate in component 1 of 14% w/w, and with hydrogen peroxide added. The composition of the components is:

**Component 1**

| Materials | Amount (g) |
|---|---|
| Calcium nitrate | 14 |
| 50%Hydrogen peroxide | 14 |
| Ethanol | 72 |

**Component 2**

| Materials | Amount (g) |
|---|---|
| Arginine | 4 |
| Lysine | 5 |
| Acetic acid | 5.5 |
| Ortho phosphoric acid | 3 |
| Ethanol | 67.5 |
| Polymer * | 15 |

| | |
|---|---|
| * Eudragit RLPO or RSPO and L100-55 in the ratio (7.5: 7.5) | |

The formulation was tested on bovine dentine tubules for 150 minutes which resulted in complete occlusion of tubules, as evidenced by CLSM. The treatment time was 30 minutes and continued five times until 150 minutes. In between each treatment, samples were washed with distilled water and to remove any unbound material from the dentine surface. The sample was then air dried for five minutes before taking the image under confocal microscope. Scanning electron microscopy (SEM) images showed no opened tubules after treatment, which confirmed the occlusion observed under CLSM.

### Example 8

This example is a model varnish composition similar to Example 7, but with the peroxide in the phosphor component.

**Component 1**

| Materials | Amount (g) |
|---|---|
| Calcium nitrate | 14 |
| Ethanol | 86 |

**Component 2**

| Materials | Amount (g) |
|---|---|
| Arginine | 4 |
| Lysine | 2 |
| 50%Hydrogen peroxide | 14 |
| Acetic acid | 4 |
| Ortho phosphoric acid | 1.7 |
| KOH | 2.5 |
| Ethanol | 71.8 |

This formulation was tested on bovine dentine tubules for three hours (multiple treatments) which resulted in complete occlusion of tubules. The treatment time was 30 minutes and continued five times 180 minutes. In between each treatment samples were washed with distilled water and then air dried for five minutes before taking the image under Confocal microscope. Complete occlusion of dentine tubules was obtained.

### Example 9

As in Example 8, with lower concentration of ortho-phosphoric acid and acetic acid.

**Component 1**

| Materials | Amount (g) |
|---|---|
| Calcium nitrate | 14 |
| Ethanol | 86 |

**Component 2**

| Materials | Amount (g) |
|---|---|
| Arginine | 4 |
| Lysine | 6 |
| 50%Hydrogen peroxide | 14 |
| Acetic acid | 2 |
| Ortho phosphoric acid | 1.5 |
| Ethanol | 72.5 |

This formulation was tested on bovine dentine tubules for four hours (multiple treatments) which resulted in complete occlusion of tubules. The treatment time was 30 minutes and continued five times 240 minutes. In between each treatment samples were washed with distilled water and then air dried for five minutes before taking a CLSM image.

### Example 10

**Component 1**

| | |
|---|---|
| Calcium Nitrate | 25 |
| Ethanol | 55 |
| RLPO | 10 |
| L100-55 | 10 |

**Component 2**

| | |
|---|---|
| 50% HP (w/w) | 40 |
| Ethanol | 26 |
| 85% Ortho phosphoric Acid | 4 |
| RLPO | 18 |
| L100-55 | 12 |

Initial studies on CLSM showed opened tubules before treatment, partially occluded tubules after 30 minutes treatment and complete tubule occlusion after 60 minutes treatment.

The formulation was also tested on enamel surface to observe any remineralisation of surface with ACP formulation.

Before treatment with formulation enamel rods were observed under SEM.
After 60 minutes treatment enamel surface appeared very smooth and the enamel rods were completely covered with the minerals from the formulation.

### Example 11

Component 1 is the same as used in example 10 and component 2 is modified with the addition of amino acid, lysine.

**Component 1**

| | |
|---|---|
| Calcium Nitrate | 25 |
| Ethanol | 55 |
| RLPO | 10 |
| L100-55 | 10 |

**Component 2**

| | |
|---|---|
| 0% HP (w/w) | 40 |
| Ethanol | 26.25 |
| 85% Ortho phosphoric Acid | 4 |
| Lysine | 4.75 |
| RLPO | 15 |
| L100-55 | 10 |

CLSM images showed opened tubules before treatment, partially occluded tubules after 30 minutes treatment and complete tubule occlusion after 60 minutes treatment.

Further, SEM images showed no opened tubules after treatment, which confirmed the occlusion observed under CLSM. The structure formed after treatment with the lysine containing formulation is different from the one without lysine.

The formulation was also tested on enamel surface to observe any remineralisation of surface with ACP formulation.

Before treatment with formulation enamel rods were observed under SEM.

After 60 minutes treatment enamel surface appeared very smooth and the enamel rods were completely covered with the minerals from the formulation.

## Claims

1. An oral healthcare system for the delivery of amorphous calcium phosphate, comprising a source of calcium ions and a source of phosphoric ions in separate components, said separate components selected from the group consisting of separate parts of a kit of parts, separate compartments of a packaging, different phases of a multiphase composition, and a combination thereof, wherein a first component is a calcium component that comprises a calcium salt and a second component is a phosphor component that comprises a source of phosphoric ions selected from the group consisting of orthophoshoric acid and phosphate salts, said components comprising a liquid carrier comprising a polar solvent, wherein either or both of said components comprise one or more amino acids.

2. A system according to claim 1,wherein the solvent is selected from the group consisting of water, acetic acid, C₂-C₁₀ mono-alcohols, C₂-C₁₀ polyols, and mixtures thereof.

3. A system according to claim 2, wherein the solvent is a mixture of ethanol and acetic acid.

4. A system according to any one of the preceding claims, wherein at least the phosphor component comprises an amino acid.

5. A system according to claim 4, wherein the amino acid comprised in the phosphor component is an acidic or neutral amino acid.

6. A system according to any one of the preceding claims, wherein the amino acids are selected from the group consisting of basic amino acids.

7. A system according to any one of the preceding claims, wherein at least one of the components comprises a gel-forming polymer.

8. A system according to any one of the preceding claims wherein the components together form a two component dental whitening system, wherein one of the components comprises at least one peroxide and the other component comprises an activator gel capable of catalysing the decomposition of the peroxide.

9. A system according to any one of the preceding claims, wherein both of the components comprise an amino acid.

10. A system according to any one of the preceding claims, wherein the amino acids are selected from the group consisting of arginine, lysine, and combinations thereof.

11. A system according to any one of the preceding claims, wherein the calcium component comprises 0.01 wt.% to 50 wt.% of the calcium salt.

12. A system according to any one of the preceding claims, wherein the phosphor component comprises 0.1 wt.% to 20 wt.% of the phosphor source.

13. A system according to any one of the preceding claims, wherein the one or more amino acid or acids are present in the composition in an amount ranging from 0.5 wt.% to 10 wt.%, preferably 2 wt.% to 8 wt %.

14. A system according to any one of the preceding claims, wherein either or both of the components comprises a source of fluoride, preferably sodium fluoride.

15. The use of one or more basic amino acids as an anti-crystallization agent for amorphous calcium phosphate formed by combining, in the oral cavity of a subject, the calcium component and the phosphor component of a system according to any one of the claims 1-14.

16. A composition comprising amorphous calcium phosphate for use in the occlusion of dental tubules and/or the deposition of remineralising agents on dental enamel, wherein the composition is applied by bringing together the components of a system according to any one of the claims 1-14 in the oral cavity of a subject.

17. Use of one or more amino acids in an oral healthcare system as an anti-crystallization agent for amorphous calcium phosphate.

18. Use according to claim 17, wherein said amino acids are one or more selected from the group consisting of arginine, lysine, and histidine.

## Patentansprüche

1. System zur Mundgesundheitspflege für die Bereitstellung von amorphem Calciumphosphat, umfassend eine Calciumionenquelle und eine Phosphorsäureionenquelle in separaten Komponenten, wobei die separaten Komponenten, ausgewählt sind aus der Gruppe, bestehend aus separaten Teilen eines Kits von Teilen, separaten Kompartimenten einer Verpackung, unterschiedlichen Phasen einer Mehrphasenzusammensetzung und einer Kombination davon, wobei eine erste Komponente eine Calciumkomponente ist, die ein Calciumsalz umfasst, und eine zweite Komponente eine Phosphorkomponente ist, die eine Phosphorsäureionenquelle umfasst, ausgewählt aus der Gruppe, bestehend aus Orthophosphorsäure- und Phosphatsalzen, wobei die Komponenten einen flüssigen Träger umfassen, umfassend ein polares Lösemittel, wobei eine oder beide der Komponenten eine oder mehrere Aminosäuren umfassen.

2. System nach Anspruch 1, wobei das Lösemittel ausgewählt ist aus der Gruppe, bestehend aus Wasser, Essigsäure, C₂-C₁₀-Monoalkoholen, C₂-C₁₀-Polyolen und Mischungen davon.

3. System nach Anspruch 2, wobei das Lösemittel eine Mischung aus Ethanol und Essigsäure ist.

4. System nach einem der vorstehenden Ansprüche, wobei mindestens die Phosphorkomponente eine Aminosäure umfasst.

5. System nach Anspruch 4, wobei die in der Phosphorkomponente umfasste Aminosäure eine saure oder neutrale Aminosäure ist.

6. System nach einem der vorstehenden Ansprüche, wobei die Aminosäuren ausgewählt sind aus der Gruppe, bestehend aus basischen Aminosäuren.

7. System nach einem der vorstehenden Ansprüche, wobei mindestens eine der Komponenten ein gelbildendes Polymer umfasst.

8. System nach einem der vorstehenden Ansprüche, wobei die Komponenten zusammen ein Zweikomponenten-Dentalaufhellungssystem bilden, wobei eine der Komponenten mindestens ein Peroxid umfasst und die weitere Komponente ein Aktivatorgel umfasst, das in der Lage ist, die Zersetzung des Peroxids zu katalysieren.

9. System nach einem der vorstehenden Ansprüche, wobei beide der Komponenten eine Aminosäure umfassen.

10. System nach einem der vorstehenden Ansprüche, wobei die Aminosäuren ausgewählt sind aus der Gruppe, bestehend aus Arginin, Lysin und Kombinationen davon.

11. System nach einem der vorstehenden Ansprüche, wobei die Calciumkomponente 0,01 Gew.-% bis 50 Gew.-% des Calciumsalzes umfasst.

12. System nach einem der vorstehenden Ansprüche, wobei die Phosphorkomponente 0,1 Gew.-% bis 20 Gew.-% der Phosphorquelle umfasst.

13. System nach einem der vorstehenden Ansprüche, wobei die eine oder die mehreren Aminosäure oder -säuren in der Zusammensetzung in einer Menge im Bereich von 0,5 Gew.-% bis 10 Gew.-%, vorzugsweise 2 Gew.-% bis 8 Gew.-% vorhanden sind.

14. System nach einem der vorstehenden Ansprüche, wobei eine oder beide der Komponenten eine Fluoridquelle umfassen, vorzugsweise Natriumfluorid.

15. Verwendung von einer oder mehreren basischen Aminosäuren als ein Antikristallisierungsmittel für amorphes Calciumphosphat, das durch Kombinieren der Calciumkomponente und der Phosphorkomponente eines Systems nach einem der Ansprüche 1 bis 14 in der Mundhöhle einer Versuchsperson gebildet wurde.

16. Zusammensetzung, umfassend amorphes Calciumphosphat zur Verwendung bei der Okklusion von Dentalkanälchen und/oder bei der Abscheidung von Remineralisationsmitteln auf Zahnschmelz, wobei die Zusammensetzung durch Zusammenbringen der Komponenten eines Systems nach einem der Ansprüche 1 bis 14 in der Mundhöhle einer Versuchsperson angewendet wird.

17. Verwendung von einer oder mehreren Aminosäuren in einem System zur Mundgesundheitspflege als ein Antikristallisierungsmittel für amorphes Calciumphosphat.

18. Verwendung nach Anspruch 17, wobei die Aminosäuren eine oder mehrere, ausgewählt aus der Gruppe, bestehend aus Arginin, Lysin und Histidin, sind.

## Revendications

1. Système de soins de santé buccaux pour la délivrance de phosphate de calcium amorphe, comprenant une source d'ions de calcium et une source d'ions phosphoriques dans des composants séparés, lesdits composants séparés étant sélectionnés dans le groupe constitué de parties séparées d'un kit de pièces, de compartiments séparés d'un emballage, de différentes phases d'une composition de phases multiples et d'une de leurs combinaisons, dans lequel un premier composant est un composant de calcium qui comprend un sel de calcium et un second composant est un composant de phosphore qui comprend une source d'ions phosphoriques sélectionnés dans le groupe constitués de l'acide orthophosphorique et de sels de phosphate, lesdits composants comprenant un véhicule liquide comportant un solvant polaire dans lequel l'un et/ou l'autre desdits composants comprend ou comprennent un ou plusieurs acides aminés.

2. Système selon la revendication 1, dans lequel le solvant est sélectionné dans le groupe constitué de l'eau, de l'acide acétique, de mono-alcools en C₂-C₁₀, de polyols en C₂-C₁₀ et de leurs mélanges.

3. Système selon la revendication 2, dans lequel le solvant est un mélange d'éthanol et d'acide acétique.

4. Système selon l'une quelconque des revendications précédentes, dans lequel au moins le composé de phosphore comprend un acide aminé.

5. Système selon la revendication 4, dans lequel l'acide aminé compris dans le composant de phosphore est un acide aminé acide ou neutre.

6. Système selon l'une quelconque des revendications précédentes, dans lequel les acides aminés sont sélectionnés dans le groupe constitué des acides aminés basiques.

7. Système selon l'une quelconque des revendications précédentes, dans lequel au moins l'un des composants comprend un polymère formateur de gel.

8. Système selon l'une quelconque des revendications précédentes, dans lequel les composants forment conjointement un système de blanchiment dentaire à deux composants, dans lequel l'un des composants comprend au moins un peroxyde et l'autre composant comprend un gel activateur capable de catalyser la décomposition du peroxyde.

9. Système selon l'une quelconque des revendications précédentes, dans lequel les deux composants comprennent un acide aminé.

10. Système selon l'une quelconque des revendications précédentes, dans lequel les acides aminés sont sélectionnés dans le groupe constitué de l'arginine, de la lysine et de leurs combinaisons.

11. Système selon l'une quelconque des revendications précédentes, dans lequel le composant de calcium comprend 0,01 % en poids à 50 % en poids du sel de calcium.

12. Système selon l'une quelconque des revendications précédentes, dans lequel le composant de phosphore comprend 0,1 % en poids à 20 % en poids de la source de phosphore.

13. Système selon l'une quelconque des revendications précédentes, dans lequel les un ou plusieurs acides aminés sont présents dans la composition en quantité allant de 0,5 % en poids à 10 % en poids, de préférence de 2 % en poids à 8 % en poids.

14. Système selon l'une quelconque des revendications précédentes, dans lequel l'un et/ou l'autre des composants comprend ou comprennent une source de fluorure, de préférence du fluorure de sodium.

15. Utilisation d'un ou plusieurs acides aminés basiques comme agent anti-cristallisation pour du phosphate de calcium amorphe formé en combinant, dans la cavité buccale d'un sujet, le composant de calcium et le composant de phosphore d'un système selon l'une quelconque des revendications 1 à 14.

16. Composition comprenant du phosphate de calcium amorphe pour utilisation dans l'occlusion de tubules dentaires et/ou le dépôt d'agents de reminéralisation sur de l'émail dentaire, dans laquelle la composition est appliquée en disposant conjointement les composants d'un système selon l'une quelconque des revendications 1 à 14 dans la cavité buccale d'un sujet.

17. Utilisation d'un ou plusieurs acides aminés dans un système de soins de santé buccaux comme agent anti-cristallisation pour du phosphate de calcium amorphe.

18. Utilisation selon la revendication 17, dans laquelle lesdits acides aminés sont un ou plusieurs acides sélectionnés dans le groupe constitué de l'arginine, de la lysine et de l'histidine.
